## Europäisches Patentamt
## European Patent Office
## Office européen des brevets

(19)

(11) Publication number: **0 054 823**
A2

(12) **EUROPEAN PATENT APPLICATION**

(21) Application number: 81110215.1

(22) Date of filing: 07.12.81

(51) Int. Cl.³: **B 02 C 18/40**

(30) Priority: **22.12.80 IT 5382580 U**

(43) Date of publication of application: **30.06.82**
**Bulletin 82/26**

(84) Designated Contracting States: **BE CH DE FR GB LI NL SE**

(71) Applicant: **BAUSANO & FIGLI S.p.A., Via Lamarmora, 9, I-10128 Torino (IT)**

(72) Inventor: **Bausano, Livio Franco, Corso Indipendenza 111, I-10086 Rivaralo Canavese (Torino) (IT)**

(74) Representative: **Modiano, Guido et al, MODIANO & ASSOCIATI S.A.S. Via Meravigli, 16, I-20123 Milan (IT)**

(54) **Solid refuse or garbage chopping and sterilizing machine, particularly for hospitals and the like.**

(57) The garbage chopping and sterilizing machine comprises a chopper unit (11) of the counter-rotating knive type and a hopper (10) for feeding trash to be processed. The chopper unit (11) is driven by a first gear motor assembly (14) and thereunder a garbage sterilization and withdrawal chamber (17) is provided comprising a trash processing and withdrawal auger (18) and a second gear motor assembly (19) for slowly rotating the auger (18), a duct-gate assembly (25, 26) being further provided for controllably feeding a steam flow in co-current relationship with the trash to be processed.

ACTORUM AG

0054823

This invention relates to a solid refuse or garbage chopping and sterilizing machine, particularly useful for hospital installations and the like, and more generally for implementing total garbage treatment plants wherein the garbage matter is reduced to bacteriologically inert waste.

More specifically the task of the invention is essentially that of providing a machine capable of carrying out, in a single step and in a fully automated manner, the mechanical process of chopping garbage or trash and simultaneously sterilizing said trash, in such a way as to deliver to the discharge end readily treatable waste material, in particular waste material suitable for bag packaging and free of any risk of bacterial infection for the operators.

Within said task it is a main object of the invention to provide a machine as indicated, which is highly efficient, reliable, and particularly easy to operate, and in particular one which can be readily disassembled for servicing or maintenance purposes.

The aforesaid task and object as well as yet other objects such as will be apparent in the course of the detailed description which follows, are achieved, according to this invention, by a solid refuse or garbage chopping and sterilizing machine, particularly for hospitals and the like, characterized in that it comprises a chopper unit of the type having knive members arranged to counter-rotate at reduced and different speeds and a hopper for feeding garbage to be processed, first gear motor means for driving said chopper unit, at least one garbage sterilization and withdrawal chamber underlying said chopper unit and comprising at least one garbage processing and withdrawal member of the rotary type, second gear motor means adapted to slowly rotate said member, and further means for controllably feeding and discharging a garbage processing steam flow, in co-current relationship with said garbage, into and out of said hopper overlying the chopper unit and respectively of said sterilization chamber.

Advantageously, the sterilization chamber is cylindrical and contains a helical auger adapted to convey the chopped trash axially along said chamber, from a feeding header or manifold to a discharge outlet, while favoring, by stirring, an intimate contact with the sterilization steam, said chamber being also equipped with condensation removal means.

Further features and advantages will be more apparent from the following detailed description, in conjunction with the accompanying drawings, where:

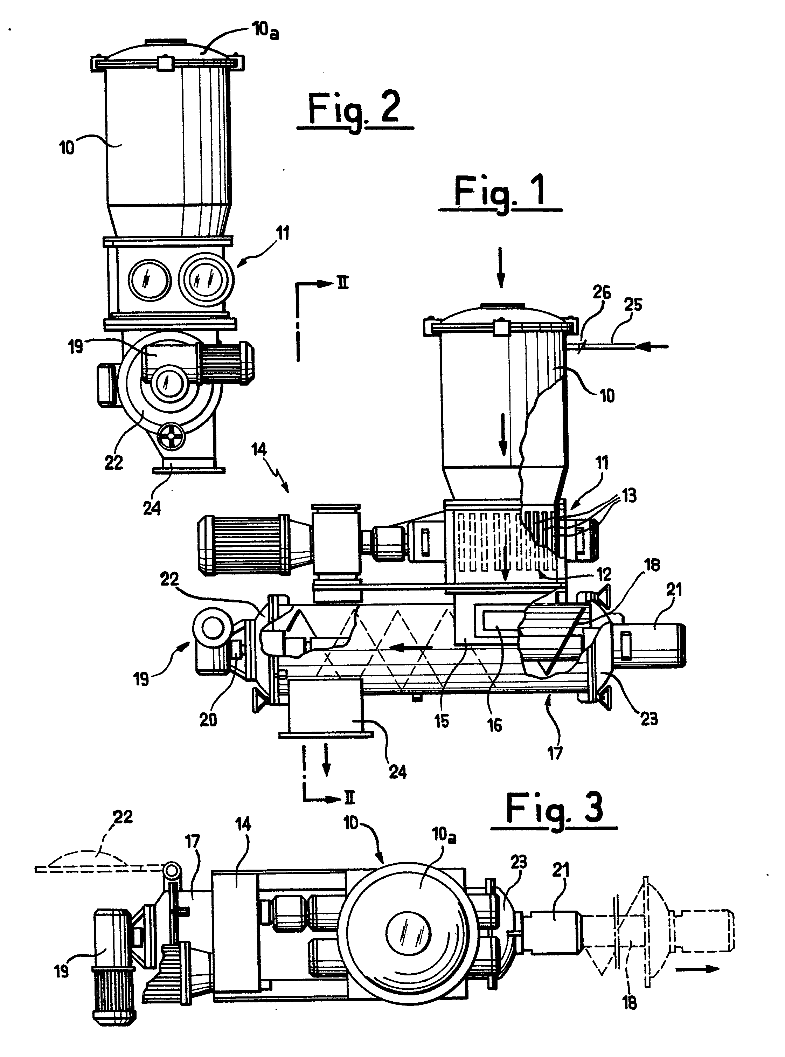

Figure 1 is a side elevation view, partly in section, of the machine according to the invention;

Figure 2 is a front elevation thereof taken in the direction of the arrows II-II of Figure 1; and

Figure 3 is a top plan view of the machine of Figure 1.

In the drawing figures, the reference numeral 10 designates a hopper whereby trash is fed by gravity to an underlying chopper unit 11. The unit 11 is advantageously of a type which comprises two counter-rotating drums 12 provided each with axially spaced apart circular knives 13, the knives intersecting one another and the drums being driven at different angular velocities. The rotary movement is imparted to the drums by a first gear motor set 14, the motion being transmitted from one drum to the other through a gear set which also provides the required step-down ratio between the angular velocities of the two drums.

The outlet of the chopper unit 11 communicates, through a header or manifold 15 having an inspection door 16, to a cylindrical chamber 17 extending with a horizontal axis and termed herein "sterilization and withdrawal chamber".The chamber 17 contains at least one handling and withdrawing member advantageously comprising a cylindrical helix auger 18 driven rotatively at an appropriate angular velocity -- proportional to the flow rate of the chopped trash discharged from the unit 11 -- by a second gear motor set 19. The auger 18 is mounted on end thrust bearings 20-21 which are carried on corresponding removable flanges 22-23 adapted to close the ends of the chamber 17 tightly.

The arrangement is such as to allow an easy withdrawal of the auger -- as is clearly shown in Figure 3 -- both for inspection and servicing purposes, as well as to remove any build-ups which may result in malfunctions. At the remote end of the chamber 17 from the manifold 15, there is provided a discharge mouth or outlet 24.

Into the hopper 10, which is closed tight by

0054823

a lid or cover $10_a$, preferably overheated steam is blown through a duct 25 having a flow control gate 26. The steam, which flows in co-current relationship with the trash material, is also passed through the chopper unit 11 and chamber 17, to be discharged through the mouth 24. The condensed portion is collected at the bottom of the chamber 17, wherefrom it is periodically removed through a condensate exhauster 27.

It will be readily appreciated that said steam, both during the chopping step and, more intensely, during the handling and withdrawal step, diffusedly contacts the chopped trash, thus bringing about an effective sterilization thereof. Upon completion of the treatment, the trash material, now in the form of crumbled and bacteriologically inert waste, is discharged from the mouth 24 and conveyed to subsequent disposal after packaging into bags, if desired. Preferably, all the machine componet parts are made of stainless steel.

Of course, the scope of this application will also cover any other modifications achieving the same result through the same inventive concept.

CLAIMS

1. A solid refuse or garbage chopping and sterilizing machine, particularly for hospitals and the like, characterized in that it comprises a chopper unit (11) of the type having knive members (13) arranged to counter-rotate at reduced and different speeds and a hopper (10) for feeding garbage to be processed, first gear motor means (14) for driving said chopper unit (11), at least one trash sterilization and withdrawal chamber (17) underlying said chopper unit (11) and comprising at least one garbage processing and withdrawal member (18) of the rotary type, second gear motor means (19) adapted to slowly rotate said member (18), and further means (25, 26) for controllably feeding and discharging a garbage processing steam flow, in co-current relationship with said garbage, into and out of said hopper (10) overlying the chopper unit (11) and respectively of said sterilization chamber (17).

2. A machine according to Claim 1, wherein said sterilization chamber (17) is cylindrical and encloses a helical auger (18) effective to convey the chopped garbage axially along said chamber (17) from a feeding header or manifold (15) to a discharge outlet (24) while favoring with a stirring action an intimate contact with the sterilization steam, said chamber (17) being provided with condensate removal means (27).

3. A machine according to the preceding claims, wherein said auger (18) is carried on end supports (20, 21) attached to removable flanges (22, 23), said removable flanges (22, 23) closing the ends of said sterilization chamber (17) tight.

4. A machine according to the preceding claims, wherein said chopper unit (11) communicates to said sterilization chamber (17) through a manifold (25) provided with an inspection door (16), and wherein the passage of said trash from said chopper unit (11) to said chamber (17) occurs by gravity.

Fig. 2

Fig. 1

Fig. 3